# EUROPÄISCHE PATENTSCHRIFT

(11) **EP 1 682 224 B1**
(45) Veröffentlichungstag und Bekanntmachung des Hinweises auf die Patenterteilung: **04.03.2009**
(21) Anmeldenummer: 04791004.7
(22) Anmeldetag: 26.10.2004
(51) Int. Cl.: A61N 5/06, H01J 61/42, F21K 2/00

(54) **STRAHLUNGSKONVERTER UND DEN KONVERTER ENTHALTENDE BESTRAHLUNGSANORDNUNG**
RADIATION CONVERTER AND IRRADIATION ARRANGEMENT CONTAINING SAID CONVERTER
CONVERTISSEUR DE RAYONNEMENT ET DISPOSITIF D'IRRADIATION CONTENANT CE CONVERTISSEUR

(30) Priorität: 03.11.2003 DE 10351706
(43) Veröffentlichungstag der Anmeldung: 26.07.2006
(73) Patentinhaber: Spectrometrix Optoelectronic Systems GmbH, 12489 Berlin (DE)
(72) Erfinder: CONRADY, Jürgen, 12623 Berlin (DE)
(74) Vertreter: Patentanwälte Bressel und Partner
(86) Internationale Anmeldenummer: PCT/EP2004/012240
(87) Internationale Veröffentlichungsnummer: WO 2005/042100

(56) Entgegenhaltungen:
- EP-A- 0 592 794
- WO-A2-01/97912
- DE-A- 10 123 926
- DE-A1- 2 910 468

## Beschreibung

Die vorliegende Erfindung betrifft einen Strahlungskonverter und eine den Konverter enthaltende Bestrahlungsordnung. Die Bestrahlungsanordnung dient insbesondere zur chronischen Behandlung von ganz oder teilweise zellvermittelten Entzündungen der Haut und der inneren Organe, von viralen und anderen infektiösen Erkrankungen, wie beispielsweise Prioneninfektionen, vor allem zur Behandlung von T-Zell-vermittelten Hauterkrankungen und Handekzemen.

Primär T-Zell-vermittelte Hauterkrankungen, wie beispielsweise atopische Dermatitis (Neurodermitis), cutanes T-Zell-Lymphom, Lichen ruber und Psiorasis beruhen auf einem Hautinfiltrat von aktivierten T-Lymphozyten des eigenen Körpers. Insbesondere von Neurodermitis sind verstärkt immer mehr Neugeborene und Kinder betroffen. Aufgrund der entzündeten Hautpartien sowie des damit verbundenen Juckreizes ist diese Erkrankung sowohl physiologisch als auch psychologisch eine schwere Belastung.

Die bisher bekannten Therapien zur Behandlung von Neurodermitis lassen sich im Wesentlichen in zwei Klassen einteilen, nämlich in die Chemotherapie und die UVA1-Lichttherapie.

Bei der Chemotherapie besteht der derzeitige Goldstandard bei der Behandlung der atopischen Dermatitis in der Glukokortikoid-Therapie. Bei dieser Behandlung kommt es sowohl nach systemischer als auch nach topischer Anwendung zu zum Teil schwerwiegenden Nebenwirkungen. Alternative Verfahren zur Behandlung der Neurodermitis beinhalten die Therapie mit stark immunmodulierenden Pharmaka, wie beispielsweise mit FK 506 oder Cyclosporin A, über deren Langzeitfolgen noch keine Erfahrungen vorliegen.

Die UVA1-Lichttherapie hat sich zur Behandlung von akuten Neurodermitis-Schüben, der Urticaria pigmentosa und der lokalisierten Sklerodermie als effektiv erwiesen. Für die UVA1-Therapie nach Meffert und die UVA1-Therapie nach Krutmann werden derzeitig zwei Gerätetypen angeboten. Die UVA1-Therapie nach Meffert arbeitet breitbandig zwischen 340 und 500 nm, die UVA1-Therapie nach Krutmann bei 340 - 400 nm.

Einen sehr guten Überblick über den Stand der Technik in der UVA1-Therapie bietet "Stellung zur Qualitätssicherung in der UVA1-Phototherapie, Fassung der Untergruppe Foto-(Chemo)Therapie und -Diagnostik der Subkommission physikalische Verfahren in der Dermatologie, Mai 1992" sowie die "Richtlinien zur Qualitätssicherung in der Foto-(Chemo)Therapie und -Diagnostik", die in Krutmann, S., Hönigsmann, H.: "Handbuch der Dermatologischen Phototherapie und -Diagnostik", Springer-Verlag, Heidelberg, Seiten 392-395, veröffentlicht ist. Als Langzeitrisiken sind dort eine vorzeitige Hautalterung und Karzinogenität aufgeführt. Aufgrund dieser Sachlage ist dort explizit aufgeführt, dass eine Anwendung von mittleren und hohen Dosen von UVA1 im Kindesalter nicht zu empfehlen ist. Damit ist jedoch gerade die größte betroffene Gruppe von Neurodermitis ausgenommen.

Bei einer Bestrahlung kommt es zu zwei Typen von DNS-Schäden, nämlich der Erzeugung von Pyrimidindimeren und oxidativen DNS-Modifikationen. Die erste Gruppe der DNS-Schäden ist über körpereigene Reparaturenzyme vergleichsweise schwer zu reparieren, und die Induktion derartiger Schäden ist karzinogen. Das Maximum dieser Schäden wird durch Bestrahlung mit einer Wellenlänge um 290 nm ausgelöst, wobei der Grad der Schädigung bei 400 nm um den Faktor 10.000 geringer und bei Wellenlängen über 425 nm nicht mehr nachweisbar ist. Diese Art der Schädigungen wird auch als CPD (Cyclobutanpyrimidin-Photodimere) bezeichnet.

Hiervon unterscheidet sich der Typ der so genannten indirekten DNS-Schädigung, der vermutlich über die Photonenanregung zellulärer Chromophore bewirkt wird. Diese Chromophore erzeugen reaktive Sauerstoffspezies, wie beispielsweise Singulett-Sauerstoff. Ein hierdurch ausgelöster DNS-Schaden (8-Hydroxyguanin) wird durch die so genannte FPG-Protein-Endonuklease repariert. Es handelt sich hierbei um vergleichsweise leichte Schäden im Bereich der DNS-Quervernetzung und nicht um DNS-Strangbrüche oder Basenverlust. Dies wurde durch Versuche mit transgenen Mäusen ohne FPG-Reparaturenzyme belegt, die dennoch keine erhöhte Tumorrate aufwiesen.

Photobiologische Wirkungen im Nicht-UV-Bereich auf Grundlage einer Wechselwirkung zwischen endogenen oder exogenen Chromophoren in der Haut gewinnen zunehmend an Bedeutung, da mit Hilfe geeigneter Strahlungsquellen therapeutische Wirkungen bei bestimmten entzündlichen Hauterkrankungen und beispielsweise Wundheilungsstörungen bei Diabetes Mellitus beeinflussbar sind.

Zur Behandlung von akuten und chronischen ganz oder teilweise zellvermittelten Entzündungen der Haut und der inneren Organe, von viralen und anderen infektiösen Erkrankungen wie HIV oder Prionenerkrankungen, Pilzinfektionen der Haut und der Schleimhäute, bakteriellen Erkrankungen der Haut und der Schleimhäute sowie Hand- und Analekzemen wird in DE 101 23 926 A1 eine Bestrahlungsanordnung mit einer Bestrahlungsquelle zur flächenhaften Bestrahlung der Behandlungsfläche beschrieben. Die Wellenlänge der emittierten Strahlung auf der Behandlungsfläche ist dabei größer als 400 nm und umfasst einen Spektralanteil im Wellenlängenbereich von 400 - 500 nm. Die Bestrahlungsanordnung umfasst Mittel zur Erzeugung von optischen Pulsen auf der Behandlungsfläche, wobei die Bestrahlungsstärke der Bestrahlungspeaks der optischen Pulse größer als 1 Watt/cm² und kleiner als 100 kW/cm² ist. Als Bestrahlungsquelle wird eine handelsübliche Xe-Blitzlampe eingesetzt.

Die von der Blitzlampe emittierte Strahlung liegt typischerweise im Bereich von 200 bis 2000 nm. Um die unerwünschte UV-Strahlung nicht auf die zu behandelnden Körperpartien gelangen zu lassen, können diese Spektralbereiche durch handelsübliche Filter herausgefiltert werden. In dem Dokument wird jedoch auch vorgeschlagen, die UV-Anteile in den gewünschten Spektralbereich zu transformieren. Zu diesem Zweck haben sich besonders aus Silikonelastomeren bestehende Folien mit anorganischen Leuchtstoffen bewährt.

Die Herstellung einer derartigen Silikonelastomerfolie ist in WO 01/21728 A1 beschrieben. Silikonelastomere eignen sich nach Angaben in diesem Dokument besser als Acrylate, transparentes PVC oder Teflon^{®} (DuPont), weil letztere nicht ausreichend thermostabil, die Silikonelastomere dagegen jedoch bis 250°C stabil sind. Letztere benötigen zudem keine Weichmacher oder andere flüchtige Substanzen, die abdampfen könnten. Zur Herstellung des Silikonelastomers wird insbesondere die radikalische Additionspolymerisationstechnik angewendet. Das Elastomer wird vorzugsweise aus einem Hydroxypolydiorganosiloxan und einem Organohydrogensiloxan in Gegenwart eines Platinkatalysators bei Raumtemperatur gebildet. Die Folie kann insbesondere auf der Außenseite des Hüllkörpers der Niederdruckentladungslampe angebracht sein, beispielsweise indem die Folie in Form eines Wechselrahmens auf dem Hüllkörper montiert oder die zu behandelnde Körperpartie mit der Folie verbandähnlich umwickelt wird. Im ersteren Falle kann die Folie auf Walzen auf- und abwickelbar gelagert sein, so dass immer ein unverbrauchter Teil der Leuchtstofffolie für eine Behandlungsanwendung zur Verfügung steht, falls der im abgewickelten Bereich enthaltene Leuchtstoff gealtert sein sollte. In diesem Falle wird die verbrauchte Leuchtstofffolie vor der Behandlung aufgewickelt.

Die Maßnahme, die Leuchtstofffolie auf eine Walze aufzuwickeln, wenn sie durch die Bestrahlung gealtert ist, ist jedoch nachteilig, weil hierzu eine große Menge dieser Folie benötigt wird und weil die Zu- und Abführung der Folie mit den Walzen apparativ aufwendige Lösungen erfordert.

Von daher liegt der vorliegenden Erfindung die Aufgabe zugrunde, eine Alterung der Folie zu vermeiden oder zumindest zu vermindern.

Diese Aufgabe wird durch den Strahlungskonverter nach Anspruch 1 und die Bestrahlungsanordnung nach Anspruch 18 gelöst. Bevorzugte Ausführungsformen der Erfindung sind in den Unteransprüchen angegeben.

Der erfindungsgemäße Strahlungskonverter wird insbesondere in Verbindung mit einer UV emittierenden Strahlungsquelle in einer erfindungsgemäßen Bestrahlungsanordnung eingesetzt. Durch den UV-Anteil in der Strahlung der Strahlungsquelle, der im Strahlungskonverter absorbiert wird, wird Licht im sichtbaren Bereich durch Fluoreszenz oder Phosphoreszenz erzeugt, dessen Wellenlänge je nach verwendeter Entladungslampe und je nach eingesetztem Strahlungskonverter im blauen, gelben, grünen oder roten Farbbereich liegt. Die Bestrahlungsanordnung wird vorzugsweise so gebildet, dass Licht mit einem großen Spektralanteil im blauen Bereich vom Strahlungskonverter emittiert wird.

Die Strahlung kann insbesondere in Lichtimpulsen emittiert werden. Hierzu können vor allem in lmpulsüberlast betriebene Mittel- und Hochdrucklampen, beispielsweise Quecksilberiodidlampen, oder eine Xe-Hochdruckblitzlampe eingesetzt werden.

Mit der erfindungsgemäßen Bestrahlungsanordnung können insbesondere Handekzeme und T-Zell-vermittelte Hauterkrankungen behandelt werden. Letztere schließen vor allem atopische Dermatitis (Neurodermitis), cutanes T-Zell-Lymphom, Lichen ruber und Psoriasis ein.

Der erfindungsgemäße Strahlungskonverter weist als wesentliches Merkmal eine Leuchtstoffschicht auf, in der die von einer Strahlungsquelle, insbesondere einer UV emittierenden Strahlungsquelle, emittierte UV-Strahlung in sichtbares, vorzugsweise blaues Licht konvertiert wird.

Es hat sich herausgestellt, dass die Alterung der Leuchtstoffschicht dann verzögert oder sogar völlig vermieden werden kann, wenn die Leuchtstoffschicht in einem Gehäuse angeordnet ist, dass von Strahlung durchstrahlbar ist, die von der UV emittierenden Strahlungsquelle emittiert wird, und das folgende Merkmale aufweist:
a) eine Vorderwand aus UV-durchlässigem Material,
b) eine Rückwand aus UV-undurchlässigem Material auf der der Vorderwand gegenüber liegenden Seite des Gehäuses,
c) wobei eine Flüssigkeitskammer zwischen der Vorderwand und der Rückwand gebildet wird,
d) die Leuchtstoffschicht zwischen der Vorder- und der Rückwand.

Bei einer Untersuchung eines Strahlungskonverters, der eine aus Silikonelastomer mit Leuchtstoff bestehende Leuchtstoffschicht aufweist, mit einer UV emittierenden Strahlungsquelle wurde gefunden, dass Alterungseffekte bereits dann merklich auftreten, wenn die auf den Konverter auftreffende Strahlungsleistungsdichte, integriert über einen Wellenlängenbereich von 260 - 1050 nm, über 400 mJ/cm² liegt.

Für die Behandlung wird der Strahlungskonverter in den Strahlengang der UV emittierenden Strahlungsquelle gebracht.

Es hat sich herausgestellt, dass die Alterung einer Leuchtstoffschicht bei Bestrahlung mit der UV emittierenden Strahlungsquelle durch deren Anordnung in dem erfindungsgemäßen Strahlungskonverter deutlich verzögert wird. So wird beobachtet, dass die Intensität der von der Schicht emittierten Strahlung, beispielsweise von blauem Licht, deutlich langsamer abnimmt als dann, wenn eine Leuchtstofffolie verwendet wird, die nicht im erfindungsgemäßen Strahlungskonverter angeordnet ist.

Das Gehäuse des Konverters besteht aus einer Vorderwand und einer Rückwand. Die Vorderwand ist die der UV emittierenden Strahlungsquelle zugewandte Wand, während die Rückwand die der Vorderwand gegenüber liegende Wand im Gehäuse ist, die von der UV emittierenden Strahlungsquelle abgewandt ist. In einer bevorzugten Ausführungsform der Erfindung bestehen sowohl die Vorderwand als auch die Rückwand aus Acrylatpolymer. Unter den Acrylatpolymeren weisen insbesondere Polymethylmethacrylate wichtige Vorteile auf, beispielsweise sehr gute optische (hohe Transparenz im sichtbaren und UV-Bereich) und thermische Eigenschaften (hohe Wärmeformbeständigkeit). Außerdem sind sie preiswert. Diese Materialien werden üblicherweise für Fenster in Sonnenliegen und anderen Beleuchtungskörpern, sofern keine zu hohe thermische Belastbarkeit gefordert wird, sowie in Gewächshäusern eingesetzt.

Von der UV emittierenden Strahlungsquelle stammende Strahlung wird zuerst durch die UV-durchlässige Vorderwand im Gehäuse geleitet und trifft nach Durchtritt durch die Flüssigkeitskammer auf die Leuchtstoffschicht. Sofern nach Durchtritt der Strahlung durch die Leuchtstoffschicht in geringem Umfange noch UV-Anteile enthalten sind, werden diese in der UV-undurchlässigen Rückwand absorbiert. Damit wird vermieden, dass UV-Strahlung die zu behandelnden Körperpartien erreicht.

Geeignete UV-durchlässige Acrylatmaterialien sind beispielsweise in EP 0 016 870 A1, EP 0 164 663 A2 und WO 02/14388 A1 offenbart. Danach kann eine auch über einen längeren Zeitraum beständige UV-Durchlässigkeit des Acrylatmaterials zum einen dadurch erreicht werden, dass die Polymerisation der zugrunde liegenden Acrylmonomere in Gegenwart eines sterisch gehinderten Amins der dort genannten Formel I durchgeführt wird. Für derartige sterisch gehinderte Amine sind vor allem Diester von 2,2,6,6-Tetramethyl-piperidyl-4-Derivaten von aliphatischen Dicarbonsäuren geeignet, insbesondere Di-(2,2,6,6-tetramethyl-piperidyl-4)-sebacat (EP 0 016 870 A1). Zum anderen kann diesen Materialien UV-Stabilität auch dadurch verliehen werden, dass der Kunststoff zusätzlich einen aliphatischen, mit Polymethylmethacrylat verträglichen und über 120°C beständigen Weichmacher enthält. Derartige Weichmacher sind beispielsweise Verbindungen mit einer oder mehreren Ester-, Ether-oder Hydroxyfunktionen, insbesondere Diethyladipat, Diethylenglykolmonomethylether und Glycerintriacetat (EP 0 164 663 A2). Schließlich kann der Polymerisationsmischung auch eine so genannte aktive Komponente zugegeben werden, um die UV-Stabilität weiter zu verbessern. Als aktive Komponenten werden insbesondere Alkohole, Wasser, Vinylverbindungen oder Butyllactat genannt (WO 02/14388 A1).

Die UV-Undurchlässigkeit der Rückwand ist dadurch erreichbar, dass dem Reaktionsgemisch vor der Polymerisation ein UV-Absorber zugegeben wird. Ein derartiger Absorber kann beispielsweise ein Bestandteil des durch Polymerisation herzustellenden Polymergerüstes sein, beispielsweise Styrol, Divinylbenzol oder eine andere aromatische Verbindung, die mit den Acrylmonomeren polymerisierbar ist. Anstelle dieser UV absorbierenden Gerüstmonomere können auch andere UV absorbierende Stoffe vorzugsweise zum Reaktionsgemisch zugegeben werden, wie beispielsweise Benzotriazol- und Benzophenon-Derivate.

Das Acrylatpolymer, aus dem die Vorderwand und die Rückwand hergestellt sind, kann entweder ein Homopolymer, ein Copolymer oder ein Blockpolymer von Acrylatmonomeren sein. Die Acrylatpolymere sind insbesondere Verbindungen, ausgewählt aus der Gruppe, umfassend Alkylacrylate und Alkylmethacrylate. Es handelt sich hierbei insbesondere um Polymethylmethacrylat sowie um die Copolymere von Methylmethacrylat mit Alkylacrylaten, insbesondere Methylacrylat und Ethylacrylat.

Die Leuchtstoffschicht kann in das Gehäuse entweder als freitragende Folie beim Zusammenbau eingebracht oder als Schicht auf eine der Wände aufgebracht werden. Da derartige Folien kommerziell verfügbar sind, ist die Verwendung einer Leuchtstofffolie vorzuziehen. Grundsätzlich kann die Schicht aber auch durch ein Auftragsverfahren auf der Wandoberfläche vor der Montage des Gehäuses gebildet werden, beispielsweise durch Aufrakeln, Siebdrucken, Vorhanggießen, Spin-Coating oder ein anderes bekanntes Verfahren, wobei eine flüssige Form des Materials für die Schicht verwendet wird, etwa eine Lösung, Emulsion oder Dispersion.

In einer bevorzugten Ausführungsform der Erfindung wird die Leuchtstoffschicht an der Rückwandfläche anliegend in das Gehäuse eingebracht. In diesem Falle werden zunächst die Vorderwand und die Flüssigkeitskammer im Gehäuse im Betrieb von der Strahlung durchstrahlt, bevor diese auf die Leuchtstoffschicht auftrifft. Grundsätzlich kann die Leuchtstoffschicht aber auch an der Vorderwand-Innenseite anliegend in das Gehäuse eingebracht werden. In diesem Falle trifft die Strahlung nach dem Durchtritt durch die Vorderwand direkt auf die Schicht, bevor sie durch die Flüssigkeitskammer durchtritt. In beiden Fällen grenzt die Leuchtstoffschicht an die Flüssigkeitskammer an. Die Alternative, bei der die Schicht an der Rückwand anliegend in das Gehäuse eingebracht wird, weist den Vorteil auf, dass die Schicht nicht so schnell altert wie dann, wenn die Leuchtstoffschicht an der Vorderwand anliegend in das Gehäuse eingebracht wird. Grundsätzlich ist auch der Fall denkbar, dass die Schicht als Folie ausgebildet ist und in der Flüssigkeitskammer freihängend aufgespannt ist, ohne mit einer der beiden Wände in Kontakt zu stehen. In diesem Falle kann es erforderlich sein, zur mechanischen Versteifung der Folie entweder einseitig oder beidseitig an die Folie anliegend ein Verstärkungsgitter vorzusehen, das in dem Strahlungskonverter stabil verankert ist und selbst eine große Steifigkeit aufweist. Dieses Gitter sollte nur einen sehr kleinen Flächenanteil der Folie abdecken, um einen merklichen Intensitätsverlust zu vermeiden.

Die Leuchtstoffschicht besteht vorzugsweise aus einem Silikonelastomer mit eingelagerten Leuchtstoffpartikeln. Durch die Ausbildung der Leuchtstoffschicht als Folie aus Silikonelastomer mit eingelagerten Leuchtstoffpartikeln können zum einen Folien ausreichender Dicke mit einer genügend hohen Leuchtstoffkonzentration hergestellt werden. Zum anderen sind die Leuchtstoffpartikel luft-und wasserfrei in dem Silikonelastomer vernetzt, so dass diese keinem Alterungsverhalten ausgesetzt sind. Wegen des hydrophoben Charakters der Silikonelastomere kann Feuchtigkeit in diese Materialien praktisch nicht eintreten, so dass die feuchteempfindlichen Leuchtstoffe nicht geschädigt werden.

Anstelle von Silikonelastomeren können aber selbstverständlich auch andere Polymere für die Leuchtstoffschicht eingesetzt werden, beispielsweise transparentes PVC und Polyfluoralkylene, insbesondere Polytetrafluorethylen (Teflon^{®}).

Die Silikonelastomerschicht wird vorzugsweise durch radikalische Additionspolymerisation in Gegenwart der einzubettenden Leuchtstoffpartikel hergestellt. Im Gegensatz zu einer Polykondensationsreaktion werden bei einer Additionspolymerisation nämlich praktisch keine schädlichen Nebenprodukte frei, beispielsweise Wasser, die die Funktion der Leuchtstoffpartikel beeinträchtigen könnten. Die Ausgangsprodukte für die Polymerisation sind insbesondere Hydroxypolydiorganosiloxane und Organohydrogensiloxane, die in Gegenwart eines Katalysators miteinander zur Reaktion gebracht werden, wobei ein vernetztes Polymerisationsprodukt entsteht. Als Hydroxypolydiorganosiloxane aus verschiedenen Polymeren werden insbesondere Stoffe mit einer Mindestviskosität von 1000 Centipoise eingesetzt, wobei es sich hierbei vorzugsweise um Hydroxypolydimethylsiloxan und dessen Copolymere mit Phenylmethylsiloxan und/oder Polymethyl-3,3,3-trifluorpropylsiloxan handelt. Das Organohydrogensiloxan enthält mindestens zwei Wasserstoffatome pro Siloxaneinheit, wobei auch in diesem Falle sowohl Homo- als auch Copolymere eingesetzt werden können. Der Katalysator kann insbesondere eine Platinverbindung, vorzugsweise Platinchlorid oder Chlorplatinsäure, sein. Durch Verwendung des Platinkatalysators kann die Polymerisationsreaktion bei Raumtemperatur durchgeführt werden, so dass die während der Polymerisation anwesenden Leuchtstoffpartikel nicht thermisch beansprucht und dadurch geschädigt werden.

Die Leuchtstoffpartikel in der Schicht sind vorzugsweise kristallin. Als besonders geeignet hat sich ein mit Europium(II)-lonen dotiertes Strontiumpyrophosphat (Sr₂P₂O₇:Eu²⁺) herausgestellt. Mit diesem Leuchtstofftyp kann UV-Strahlung in blaues Licht konvertiert werden. Um die UV-Strahlung auch in sichtbares Licht mit anderer spektraler Verteilung umzuwandeln oder auch aus anderen Gründen, können auch andere Leuchtstoffe eingesetzt werden. In einer besonders geeigneten Ausführungsform ist die Leuchtstoffschicht so ausgebildet, dass sie in verschiedenen Bereichen unterschiedliche Leuchtstoffpartikel enthält. Dies ist dann von Vorteil, wenn der zu behandelnde Patient gleichzeitig in unterschiedlichen Bereichen mit Licht unterschiedlicher spektraler Zusammensetzung bestrahlt werden soll. Mit derartigen Anordnungen können Körperpartien in schnellem Wechsel auch mit Licht unterschiedlicher spektraler Verteilung bestrahlt werden.

Die Leuchtstoffschicht hat vorzugsweise eine Dicke im Bereich von 10 bis 800 µm, besonders bevorzugt von 100 bis 600 µm, wobei die Dicke einer der Parameter für die Optimierung des Wirkungsgrades der Strahlungserzeugung ist. Der Wirkungsgrad hängt im Wesentlichen von der Flächendichte des Leuchtstoffes in der Schicht ab. Diese sollte im Bereich von 1 bis 20 mg/cm² liegen. Besonders vorteilhaft sind Flächendichten im Bereich von 3 bis 6 mg/cm²_{.}

Einen wesentlichen Einfluss auf die Wirksamkeit der Strahlungserzeugung haben die geometrischen Verhältnisse im Strahlungskonverter: Werden die Gehäusewände beispielsweise nicht in Form von ebenen Platten sondern gekrümmt ausgebildet, so wird nur eine sehr geringe Strahlungsleistung im sichtbaren Bereich erreicht. Die Ursache hierfür ist nicht bekannt. Werden die Vorderwand und die Rückwand dagegen als Platten ausgebildet, so wird eine wesentlich verbesserte Strahlungsleistung erreicht. Eine optimierte Abstrahlung wird dann ermöglicht, wenn die Vorderwand und die Rückwand planparallele Flächen zueinander bilden. Die Dicke der Flüssigkeitskammer liegt vorzugsweise im Bereich von 0,5 - 5 mm. Sie beträgt besonders bevorzugt 2 mm.

Die Flüssigkeitskammer im Strahlungskonverter wird mit mindestens einer Kühlflüssigkeit gefüllt. Als Kühlflüssigkeiten werden vorzugsweise Substanzen verwendet, die ausgewählt sind aus der Gruppe, umfassend Silikonöle und Wasser, wobei sich Silikonöle als noch geeigneter als Wasser erwiesen haben. Im Falle von Wasser als Kühlflüssigkeit sinkt die Strahlungsleistung in Ruhephasen, d.h. wenn keine Strahlung auf den Konverter fällt, mit der Zeit ab. Es ist beobachtet worden, dass sich in diesen Ruhephasen Gasbläschen und eine Trübung in der Flüssigkeit bilden. Um die Gasbläschen zu entfernen, kann ein viskoelastischer Bereich in mindestens einer der beiden Wände vorgesehen sein, beispielsweise ein Gummistopfen, der sich durch die gesamte Dicke dieser Wand hindurch erstreckt. Die Gasbläschen können dann mit einer Spritze, die durch den Stopfen gestochen wird, abgezogen werden.

Die Abstimmung der Brechungsindizes der Kühlflüssigkeit, des Materials für die Leuchtstoffschicht und der Materialien für die Wände aufeinander ist für die Intensität der aus dem Strahlungskonverter austretenden Strahlung im sichtbaren Bereich sehr wichtig:

Da die in der Leuchtstofffolie erzeugte Strahlung isotrop abgestrahlt wird, wird etwa die Hälfte der Strahlung auch in Richtung der UV emittierenden Strahlungsquelle zurück gestrahlt. Um diesen Anteil zugunsten der in Richtung der zu behandelnden Körperpartie abgestrahlten Strahlung zu vermindern, können die Brechungsindizes der genannten Materialien so aufeinander abgestimmt werden, dass zumindest ein Teil der Strahlung aus der Leuchtstoffschicht in Richtung der Strahlungsquelle durch Totalreflexion nicht austreten kann und ein entsprechender Austritt in Richtung der Körperpartie nicht durch Totalreflexionseffekte behindert wird. Wird die Schicht beispielsweise an der Rückwand anliegend in das Gehäuse eingebracht, so sollte der Brechungsindex des Materials der Rückwand größer sein als der der Leuchtstoffschicht und letzterer wiederum größer als der der Kühlflüssigkeit. Die in diesem Falle von der UV emittierenden Strahlungsquelle kommende Strahlung kann unabhängig von den jeweiligen Brechungsindizes problemlos durch die Vorderwand in die Kühlflüssigkeit und von dort in die Leuchtstoffschicht gelangen, da diese Strahlung gerichtet ist, so dass Totalreflexion an den Grenzflächen zwischen der Vorderwand und der Kühlflüssigkeit und zwischen der Kühlflüssigkeit und der Leuchtstoffschicht durch geeignete Strahlungsführung, insbesondere durch im Wesentlichen senkrechtes Bestrahlen des Strahlungskonverters mit der UV-Strahlung, vermieden werden kann.

In gleicher Weise können die Brechungsindizes der genannten Materialien auch dann in geeigneter Weise aufeinander abgestimmt werden, wenn die Leuchtstoffschicht an der Vorderwand anliegend in das Gehäuse eingebracht ist oder wenn die Schicht in Form einer Folie frei hängend in der Kühlflüssigkeit aufgespannt ist. In letzterem Falle kann die Intensität der aus dem Strahlungskonverter austretenden Strahlung auch durch geeignete Wahl der Kühlflüssigkeiten in den beiden durch die Folie gebildeten Flüssigkeitskammern dadurch optimiert werden, dass unterschiedliche Flüssigkeiten mit geeigneten Brechungsindizes im Verhältnis zu denen der Materialien der Leuchtstoffschicht und der beiden Wände ausgewählt werden.

Die Vorderwand und die Rückwand können zum flüssigkeitsdichten Abschluss der Flüssigkeitskammer in deren Randbereichen stoffschlüssig miteinander verbunden werden. Beispielsweise kann die eine der beiden Wände einen randseitigen Randvorsprung aufweisen, auf den die andere Wand aufgesetzt wird. An den Verbindungsflächen werden die beiden Wände dann miteinander verbunden, beispielsweise verklebt, vorzugsweise mit einem Acrylkleber. Je nach Art der Leuchtstoffschicht kann sich diese auch in den Bereich der Verbindungsflächen hinein erstrecken, so dass die beiden Wände über die Schicht miteinander verbunden werden.

Um zu vermeiden, dass die Verbindungsstellen von der Strahlung beeinträchtigt werden, kann die Vorderwand zu deren Abschattung in den Randbereichen mit einer Maske versehen werden, so dass die Strahlung nicht direkt auf die Verbindungsstellen fällt. Die Maske kann entweder ein gegen die Strahlung beständiger Schutzlack sein, der strahlungsundurchlässig ist, oder ein Abdeckrahmen, der auf das Strahlungskonvertergehäuse aufgeklemmt wird.

Weiterhin kann eine Wechselhalterung für das Gehäuse vorgesehen sein. Dadurch wird ein leichter Austausch oder Ersatz des Gehäuses durch ein neues Gehäuse ermöglicht, etwa wenn die Wirksamkeit der Leuchtstoffschicht durch die Strahlung bereits beeinträchtigt ist und die Schicht durch eine andere ausgetauscht werden muss oder wenn eine Leuchtstoffschicht mit anderen Leuchtstoffen zur Erzeugung einer Strahlung mit einem anderen Spektralbereich oder mit anderen Bestrahlungsstärken eingesetzt werden soll. Die Wechselhalterung kann vorteilhaft unmittelbar mit der UV emittierenden Strahlungsquelle verbunden sein. Dadurch wird eine kompakte Bauweise erreicht. Außerdem ist der Konverter dadurch in unmittelbarer Nähe zur Strahlungsquelle angeordnet, so dass der Konverter auch eine geringe Baugröße haben kann, da der Strahlungsquerschnitt in der Nähe der Strahlungsquelle kleiner ist als in größerer Entfernung von dieser.

Für bestimmte Behandlungsarten kann es vorteilhaft sein, die bestrahlten Körperpartien topisch mit Sauerstoff zu spülen. Zur Wirkung der Sauerstoffbehandlung wird auch auf DE 101 23 926 A1 verwiesen. Dadurch wird der Sauerstoffgehalt in den zu behandelnden Körperpartien erhöht, was wiederum die Bildung von therapeutisch wirksamen reaktiven O₂-Spezies erhöht. Für diesen Fall kann der Strahlungskonverter eine glockenförmige Ausbildung an der Rückwand haben. Der Konverter wird in diesem Falle auf die zu behandelnde Körperpartie aufgesetzt, so dass sich zwischen der glockenförmigen Rückwand und der Körperpartie ein Hohlraum bildet, der mit Sauerstoff geflutet werden kann. Für diesen Zweck ist an der glockenförmigen Rückwand ein Zuführstutzen für Sauerstoff vorgesehen. Durch diese Ausführungsform wird ebenfalls eine sehr kompakte Bauform erreicht. Falls der Strahlungskonverter zusätzlich mit einem Kühlkreislauf für die Kühlflüssigkeit ausgebildet ist, können die UV emittierende Strahlungsquelle und der Strahlungskonverter in der kompakten Bauform über die Glocke direkt auf die zu behandelnde Körperpartie aufgesetzt werden, ohne dass zu befürchten ist, dass die Körperpartie durch übermäßige Wärmeentwicklung geschädigt wird.

Die nachfolgenden Figuren dienen zur Veranschaulichung der Erfindung. Die Figuren zeigen:
- Fig. 1a:: einen seitlichen Schnitt durch das Gehäuse eines Strah- lungskonverters,
- Fig. 1b:: eine Draufsicht auf das Gehäuse des Strahlungskonverters,
- Fig. 2:: einen seitlichen Schnitt durch eine Bestrahlungsanordnung.

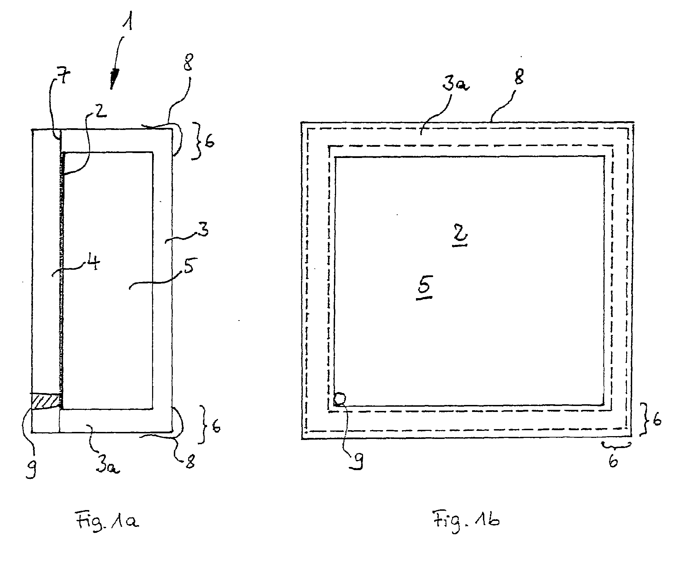

In Fig. 1a ist ein seitlicher Schnitt durch das Gehäuse 1 eines Strahlungskonverters gezeigt. Fig. 1b zeigt eine Draufsicht auf dieses Gehäuse 1. Das Gehäuse 1 besteht aus einer UV-durchlässigen Vorderwand 3 und einer UV-undurchlässigen Rückwand 4. Die beiden Wände bestehen aus Polymethylmethacrylat. Die Vorderwand 3 weist in den Randbereichen 6 einen Randvorsprung 3a auf, die sich über die gesamten Randbereiche 6 erstreckt. Die Vorderwand 3 liegt mit den Stirnflächen des Randvorsprungs 3a auf den entsprechenden Randbereichen 6 der Rückwand 4 auf. Dort sind die beiden Wände 3,4 miteinander flüssigkeits- und gasdicht verklebt. Durch die dadurch geschaffene stoffschlüssige Verbindung an der Verbindungsstelle 7 der Vorderwand 3 mit der Rückwand 4 wird eine Kammer 5 gebildet. Diese Kammer 5 ist mit Silikonöl, beispielsweise mit Baysilone^{®}-Öl-M (Bayer), gefüllt.

Auf der Rückwand 4 ist im Bereich der Kammer 5 eine Leuchtstofffolie 2 befestigt, die aus einem Silikonelastomer und Leuchtstoffpartikeln aus Sr₂P₄O₇:Eu2⁺ besteht, um vorzugsweise eine gewünschte Emission im Bereich von 400 bis 500 nm zu erhalten.

In den Randbereichen 6 ist eine Maske 8 auf die Vorderwand 3 aufgeklemmt. Diese Maske 8 ist als profiliertes Federblech ausgebildet, das die Randbereiche zumindest soweit überspannt, dass das Gehäuse 1 im Bereich der Verbindungsstellen 7 gegen Strahlung, die auf das Gehäuse 1 trifft, abgeschattet wird.

Das profilierte Blech 8 ist an den äußeren Rändern so umgebogen, dass es die Vorderwand 3 klemmend umgreifen kann, wenn es auf diese aufgesteckt ist.

Um Gasbläschen aus der Kammer 5 zu entfernen, ist außerdem ein viskoelastischer Bereich 9 (eingeklebter Stopfen) in der Rückwand 4 vorgesehen, durch den beispielsweise eine Spritze geführt werden kann, um das Gas abzuziehen.

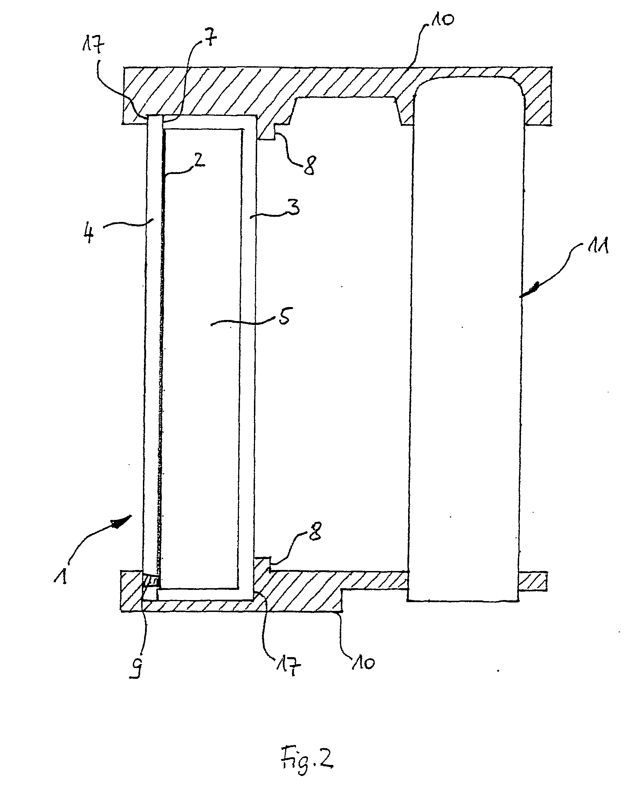

Fig. 2 zeigt eine erfindungsgemäße Bestrahlungsanordnung im Querschnitt von der Seite gesehen. Das Gehäuse 1 ist mit dem in den Fig. 1a und 1b gezeigten Gehäuse 1 weitgehend identisch. Daher betreffen gleiche Bezugsziffern im Wesentlichen auch gleiche Elemente. Auf die entsprechende Figurenbeschreibung wird Bezug genommen.

Im Unterschied zu der in den Fig. 1 a und 1 b gezeigten Ausführungsform ist die Maske 8 in diesem Falle in die Wechselhalterung 10 integriert. Die Wechselhalterung 10 weist zu diesem Zweck in den Randbereichen der Vorderwand Vorsprünge auf, die die Verbindungsstellen 7 gegen die Strahlung abschatten.

Das Gehäuse 1 wird zusammen mit einer UV emittierenden Strahlungsquelle 11 in einer Wechselhalterung 10 gehalten. Das Gehäuse ist hierzu in zur Seite hin offenen Führungsschienen 17 eingeschoben und kann somit leicht ausgetauscht werden. Die UV emittierende Strahlungsquelle 11 ist in der Wechselhalterung 10 ebenfalls austauschbar gehalten, wobei die hierzu vorgesehenen Halteelemente in der Fig. 2 nicht gezeigt sind. Es ist lediglich schematisch angedeutet, dass die Strahlungsquelle 11 in der Wechselhalterung 10 befestigt ist.

### Bezugszeichenliste:

- 1: Gehäuse des Strahlungskonverters
- 2: Leuchtstofffolie
- 3: Vorderwand des Gehäuses 1 mit dem Randvorsprung 3a (Fig. 2)
- 4: Rückwand des Gehäuses 1
- 5: Flüssigkeitskammer
- 6: Randbereiche der Vorderwand 3 und der Rückwand 4
- 7: Verbindungsstelle
- 8: Maske
- 9: Viskoelastischer Bereich (Stopfen)
- 10: Wechselhalterung
- 11: UV emittierende Strahlungsquelle

## Patentansprüche

1. Strahlungskonverter für eine UV emittierende Strahlungsquelle, umfassend eine Leuchtstoffschicht
**dadurch gekennzeichnet, dass**
die Leuchtstoffschicht (2) in einem von von der Strahlungsquelle emittierter UV-Strahlung durchstrahlbaren Gehäuse (1) angeordnet ist, das
a. eine Vorderwand (3) aus UV-durchlässigem Material und
b. eine Rückwand (4) aus UV-undurchlässigem Material auf der der Vorderwand (3) gegenüber liegenden Seite des Gehäuses (1) aufweist,
c. wobei eine Flüssigkeitskammer (5) zwischen der Vorderwand (3) und der Rückwand (4) gebildet wird und
d. die Leuchtstoffschicht (2) zwischen der Vorderwand (3) und der Rückwand (4) angeordnet ist

2. Strahlungskonverter nach Anspruch 1, **dadurch gekennzeichnet, dass** die Leuchtstoffschicht (2) in Form einer Folie vorliegt.

3. Strahlungskonverter nach einem der vorstehenden Ansprüche, **dadurch gekennzeichnet, dass** die Leuchtstoffschicht (2) auf der Rückwand (4) aufliegt und an die Flüssigkeitskammer (5) angrenzt.

4. Strahlungskonverter nach einem der vorstehenden Ansprüche, **dadurch gekennzeichnet, dass** die Leuchtstoffschicht (2) aus Silikonelastomer mit eingelagerten Leuchtstoffpartikeln besteht.

5. Strahlungskonverter nach Anspruch 4, **dadurch gekennzeichnet, dass** die Leuchtstoffschicht (2) in verschiedenen Bereichen unterschiedliche Leuchtstoffpartikel enthält.

6. Strahlungskonverter nach einem der Ansprüche 4 und 5, **dadurch gekennzeichnet, dass** die eingelagerten Leuchtstoffpartikel aus Sr₂P₂OEu²⁺ bestehen.

7. Strahlungskonverter nach einem der vorstehenden Ansprüche, **dadurch gekennzeichnet, dass** das Silikonelastomer durch radikalische Additionspolymerisation hergestellt ist.

8. Strahlungskonverter nach einem der vorstehenden Ansprüche, **dadurch gekennzeichnet, dass** die Vorderwand (3) und die Rückwand (4) planparallele Flächen bilden.

9. Strahlungskonverter nach einem der vorstehenden Ansprüche, **dadurch gekennzeichnet, dass** die Flüssigkeitskammer (5) mit mindestens einer Kühlflüssigkeit gefüllt ist.

10. Strahlungskonverter nach Anspruch 9, **dadurch gekennzeichnet, dass** die mindestens eine Kühlflüssigkeit ausgewählt ist aus der Gruppe umfassend Silikonöle und Wasser.

11. Strahlungskonverter nach einem der vorstehenden Ansprüche, **dadurch gekennzeichnet, dass** die Vorderwand (3) und die Rückwand (4) aus Acrylatpolymer bestehen.

12. Strahlungskonverter nach Anspruch 11, **dadurch gekennzeichnet, dass** das Acrylatpolymer ein Homo-, Co- oder Blockpolymer von Acrylatmonomeren ist, ausgewählt aus der Gruppe umfassend Alkylacrylate und Alkylmethacrylate.

13. Strahlungskonverter nach einem der vorstehenden Ansprüche, **dadurch gekennzeichnet, dass** die Vorderwand (3) und die Rückwand (4) zum flüssigkeitsdichten Abschluss der Flüssigkeitskammer (5) in deren Randbereichen (6) stoffschlüssig miteinander verbunden sind.

14. Strahlungskonverter nach Anspruch 13, **dadurch gekennzeichnet, dass** die stoffschlüssige Verbindung eine Verklebung ist.

15. Strahlungskonverter nach einem der vorstehenden Ansprüche, **dadurch gekennzeichnet, dass** die Vorderwand (3) zur Abschattung von Verbindungsstellen (7) der beiden Wände in den Randbereichen (6) mit einer Maske (8) versehen ist.

16. Strahlungskonverter nach einem der vorstehenden Ansprüche, **dadurch gekennzeichnet, dass** ein viskoelastischer Bereich (9) in mindestens einer der beiden Wände (3,4) vorgesehen ist, der sich durch die gesamte Dicke dieser Wand (4) hindurch erstreckt.

17. Strahlungskonverter nach einem der vorstehenden Ansprüche, **dadurch gekennzeichnet, dass** er außerdem eine Wechselhalterung (10) für das Gehäuse (1) aufweist.

18. Bestrahlungsanordnung, umfassend eine UV emittierende Strahlungsquelle (11) und eine Leuchtstoffschicht (2),
**dadurch gekennzeichnet, dass**
der Strahlungskonverter nach einem der Ansprüche 1 - 17 im Strahlengang der UV emittierenden Strahlungsquelle (11) angeordnet ist.

19. Bestrahlungsanordnung nach Anspruch 18, **dadurch gekennzeichnet, dass** der Strahlungskonverter in einer Wechselhalterung (10) gehalten wird, die mit der UV emittierenden Strahlungsquelle (11) verbunden ist.

## Claims

1. Radiation converter for a radiation source that emits UV, the said radiation converter comprising a luminous material layer, **characterised in that** the luminous material layer (2) is disposed in a housing (1) that can be penetrated by the UV radiation that is emitted by the radiation source, the said housing
a. having a front wall (3) produced from material that is transparent to UV and
b. a rear wall (4) produced from material that is not transparent to UV on the side of the housing (1) situated opposite the front wall (3),
c. wherein a liquid chamber (5) is formed between the front wall (3) and the rear wall (4) and
d. the luminous material layer (2) is disposed between the front wall (3) and the rear wall (4).

2. Radiation converter according to claim 1, **characterised in that** the luminous material layer (2) is in the form of a foil.

3. Radiation converter according to one of the preceding claims, **characterised in that** the luminous material layer (2) is on the rear wall (4) and adjoins the liquid chamber (5).

4. Radiation converter according to one of the preceding claims, **characterised in that** the luminous material layer (2) is produced from silicone elastomer with inserted luminous material particles,

5. Radiation converter according to claim 4, **characterised in that** the luminous material layer (2) includes different luminous material particles in various regions.

6. Radiation converter according to one of claims 4 and 5, **characterised in that** the inserted luminous material particles are produced from Sr₂P₂O₇:EU^{2+.}

7. Radiation converter according to one of the preceding claims, **characterised in that** the silicone elastomer is produced by radical addition polymerisation.

8. Radiation converter according to one of the preceding claims, **characterised in that** the front wall (3) and the rear wall (4) form plane-parallel surfaces.

9. Radiation converter according to one of the preceding claims, **characterised in that** the liquid chamber (5) is filled with at least one cooling liquid.

10. Radiation converter according to claim 9, **characterised in that** the at least one cooling liquid is selected from the group comprising silicone oils and water.

11. Radiation converter according to one of the preceding claims, **characterised in that** the front wall (3) and the rear wall (4) are produced from acrylic polymer.

12. Radiation converter according to claim 11, **characterised in that** the acrylic polymer is a homopolymer, copolymer or block polymer of acrylic monomers, selected from the group comprising alkyl acrylate and alkyl methacrylate.

13. Radiation converter according to one of the preceding claims, **characterised in that** the front wall (3) and the rear wall (4) are interconnected in a positively bonded manner for the liquid-proof closure of the liquid chamber (5) in its end regions (6).

14. Radiation converter according to claim 13, **characterised in that** the positively bonded connection is an adhesive bond.

15. Radiation converter according to one of the preceding claims, **characterised in that** the front wall (3) is provided with a mask (8) for shadowing connection points (7) of the two walls in the edge regions (6).

16. Radiation converter according to one of the preceding claims, **characterised in that** a viscoelastic region (9) is provided in at least one of the two walls (3, 4), the said region extending through the entire thickness of the said wall (4).

17. Radiation converter according to one of the preceding claims, **characterised in that** the said radiation converter also has an interchangeable holder (10) for the housing (1).

18. Irradiation arrangement, comprising a radiation source (11) that emits UV and a luminous material layer (2), **characterised in that** the radiation converter is disposed according to one of claims 1 - 17 in the ray path of the radiation source (11) that emits UV.

19. Irradiation arrangement according to claim 18, **characterised in that** the radiation converter is retained in an interchangeable holder (10), which is connected to the radiation source (11) that emits UV.

## Revendications

1. Convertisseur de rayonnement pour une source de rayonnement émettant des UV, comprenant une couche de substance luminescente,
**caractérisé en ce que**
la couche de substance luminescente (2) est disposée dans un boîtier (1) pouvant être traversé par le rayonnement UV émis de la source de rayonnement qui présente
a. une paroi frontale (3) en matériau perméable aux UV, et
b. une paroi arrière (4) en matériau non perméable aux UV sur le côté se trouvant opposé à la paroi frontale (3) du boîtier (1),
c. une chambre de fluide (5) étant formée entre la paroi frontale (3) et la paroi arrière (4) et
d. la couche de substance luminescente (2) étant disposée entre la paroi frontale (3) et la paroi arrière (4).

2. Convertisseur de rayonnement selon la revendication 1, **caractérisé en ce que** la couche de substance luminescente (2) se présente sous la forme d'une feuille.

3. Convertisseur de rayonnement selon l'une quelconque des revendications précédentes, **caractérisé en ce que** la substance luminescente (2) se trouve sur la paroi arrière (4) et est adjacente à la chambre de fluide (5).

4. Convertisseur de rayonnement selon l'une quelconque des revendications précédentes, **caractérisé en ce que** la couche de substance fluorescente (2) est constituée d'élastomère de silicone comportant des particules luminescentes incorporées.

5. Convertisseur de rayonnement selon la revendication 4, **caractérisé en ce que** la couche de substance luminescente (2) contient dans différentes zones, des particules luminescentes différentes.

6. Convertisseur de rayonnement selon l'une quelconque des revendications 4 et 5, **caractérisé en ce que** les particules fluorescentes incorporées sont constituées de Sr₂P₂O₇:Eu²⁺ .

7. Convertisseur de rayonnement selon l'une quelconque des revendications précédentes, **caractérisé en ce que** l'élastomère de silicone est produit par polymérisation radicalaire par addition.

8. Convertisseur de rayonnement selon l'une quelconque des revendications précédentes, **caractérisé en ce que** la paroi frontale (3) et la paroi arrière (4) forment des surfaces à plan parallèle.

9. Convertisseur de rayonnement selon l'une quelconque des revendications précédentes, **caractérisé en ce que** la chambre de fluide (5) est remplie d'au moins un liquide de refroidissement.

10. Convertisseur de rayonnement selon la revendication 9, **caractérisé en ce qu'**au moins un liquide de refroidissement est choisi dans le groupe comprenant les huiles de silicone et l'eau.

11. Convertisseur de rayonnement selon l'une quelconque des revendications précédentes, **caractérisé en ce que** la paroi frontale (3) et la paroi arrière (4) sont constituées de polymère acrylate.

12. Convertisseur de rayonnement selon la revendication 11, **caractérisé en ce que** le polymère acrylate est un homopolymère, un copolymère ou un polymère en blocs de monomères acrylate, choisi dans le groupe comprenant les alkylacrylates et les alkylméthacrylates.

13. Convertisseur de rayonnement selon l'une quelconque des revendications précédentes, **caractérisé en ce que** la paroi frontale (3) et la paroi arrière (4) sont reliées l'une à l'autre de façon adhésive en une fermeture étanche aux liquides de la chambre de fluide (5), au niveau de leurs pourtours (6).

14. Convertisseur de rayonnement selon la revendication 13, **caractérisé en ce que** le raccordement adhésif est un collage.

15. Convertisseur de rayonnement selon l'une quelconque des revendications précédentes, **caractérisé en ce que** la paroi frontale (3) est dotée d'un masque (8) pour l'obscurcissement de points de raccordement (7) des deux parois au niveau des pourtours (6).

16. Convertisseur de rayonnement selon l'une quelconque des revendications précédentes, **caractérisé en ce qu'**une zone viscoélastique (9) est prévue dans au moins l'une des deux parois (3, 4), qui s'étend sur l'épaisseur entière de cette paroi (4).

17. Convertisseur de rayonnement selon l'une quelconque des revendications précédentes, **caractérisé en ce qu'**il présente en outre un support amovible (10) pour le boîtier (1).

18. Dispositif d'irradiation, comprenant une source de rayonnement émettant des UV (11) et une couche de substance luminescente (2), **caractérisé en ce que**
le convertisseur de rayonnement est disposé selon l'une quelconque des revendications 1 à 17, dans le trajet de rayons de la source de rayonnement émettant des UV (11).

19. Dispositif d'irradiation selon la revendication 18, **caractérisé en ce que** le convertisseur de rayonnement est maintenu dans un support amovible qui est relié à la source d'irradiation émettant des UV (11).
